# EUROPEAN PATENT APPLICATION

(11) **EP 3 059 230 A1**
(43) Date of publication of application: **24.08.2016**
(21) Application number: 15155418.5
(22) Date of filing: 17.02.2015
(51) Int. Cl.: C07D 301/12

(54) **Method for the epoxidation of propene with hydrogen peroxide**

(71) Applicant: Evonik Degussa GmbH, 45128 Essen (DE)
(72) Inventor: Stock, Jürgen, 60599 Frankfurt (DE); Rein, Daniel, 63607 Wächtersbach (DE); Schallenberg, Jörg, 46286 Dorsten (DE); Thiele, Georg Friedrich, 61169 Friedberg (DE)

(57) **Abstract**

The method for the epoxidation of propene comprises continuously reacting propene with hydrogen peroxide in the presence of a homogeneous epoxidation catalyst in a reaction mixture comprising an aqueous liquid phase and an organic liquid phase using a loop reactor with mixing of the liquid phases, withdrawing aqueous phase from the loop reactor and extracting it by liquid-liquid-extraction using propene as extractant to provide an extract comprising propene and propene oxide.

## Description

### Field of the invention

The invention relates to a method for the epoxidation of propene with hydrogen peroxide in the presence of a homogeneous epoxidation catalyst where the reaction is carried out in a reaction mixture comprising an aqueous liquid phase and an organic liquid phase.

### Background of the invention

Methods for the epoxidation of an olefin with hydrogen peroxide using a homogeneous water soluble manganese complex as epoxidation catalyst are known from D. E. De Vos et al., Tetrahedron Letters 39 (1998) 3221-3224 and from US 5,329,024.

WO 2011/063937 discloses epoxidation of propene in an aqueous reaction medium with a water soluble manganese complex as epoxidation catalyst. The reaction product propene oxide may be stripped with propene at low pressure or may form an organic phase with liquefied propene at high pressure. WO 2011/107188 teaches using a loop reactor for this reaction.

US 5,274,140 discloses epoxidation of propene with hydrogen peroxide in the presence of a catalyst system comprising a heteropolytungstate as epoxidation catalyst and tricaprylmethylammonium chloride as phase transfer catalyst. The reaction is carried out with 1,2-dichloromethane as solvent.

When propene is epoxidized with hydrogen peroxide and a homogeneous catalyst in a two phase reaction mixture, the aqueous phase will contain a considerable amount of the product propene oxide. Recovery of propene oxide by distillation leads to hydrolysis of propene oxide when the distillation is carried out at the acidic pH at which the epoxidation is usually performed.

### Summary of the invention

It has now been found that propene oxide can be recovered from the aqueous phase of a two phase epoxidation reaction mixture with low energy requirement and little hydrolysis of propene oxide by extraction with propene.

When a water soluble epoxidation catalyst comprising a manganese complex is used and extraction is carried out in an extraction column, the extraction column may serve as an additional reactor for increasing hydrogen peroxide conversion to propene oxide.

Subject of the invention is therefore a method for the epoxidation of propene, comprising continuously reacting propene with hydrogen peroxide in the presence of a homogeneous epoxidation catalyst, the reaction being carried out in a reaction mixture comprising an aqueous liquid phase and an organic liquid phase using a loop reactor with mixing of the liquid phases, where aqueous phase withdrawn from the loop reactor is extracted by liquid-liquid-extraction using propene as extractant to provide an extract comprising propene and propene oxide.

The homogeneous epoxidation catalyst preferably comprises a water soluble manganese complex and the extraction is preferably carried out in an extraction column.

### Brief description of drawings

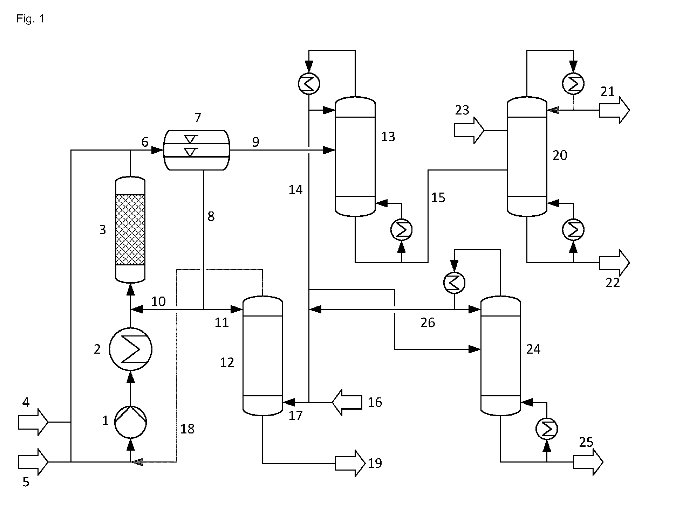

Fig. 1 shows a preferred embodiment of the method of the invention using both propene starting material and non-reacted propene recovered from the reaction mixture as extractant in an extraction column operated in counter current flow.

### Detailed description of the invention

In the method of the invention propene is reacted with hydrogen peroxide in the presence of a homogeneous epoxidation catalyst in a reaction mixture comprising an aqueous liquid phase and an organic liquid phase.

Hydrogen peroxide can be used as an aqueous solution, preferably containing from 20 to 75 % by weight hydrogen peroxide and most preferably from 40 to 70 % by weight. Preferably, an aqueous hydrogen peroxide solution prepared by an anthraquinone process is used. A crude hydrogen peroxide solution as obtained in the extraction step of the anthraquinone process may be used in the method of the invention.

In one embodiment of the invention, the homogeneous epoxidation catalyst is a water soluble epoxidation catalyst comprising a manganese complex. The manganese complex preferably comprises at least one polydentate ligand which preferably coordinates through nitrogen atoms, most preferably through tertiary amino groups. The manganese complex may be a mononuclear complex of formula [LMnXₘ]Yₙ, a dinuclear complex of formula [LMn(µ-X)ₘMnL]Yₙ or a polynuclear complex of formula [LₚMnₚ(µ-X)ₘ]Yₙ, where L is a polydentate ligand, X is a coordinating species, µ-X is a bridging coordinating species, Y is a non-coordinating counter ion, m is 1, 2 or 3, n is an integer providing for the charge neutrality of the complex, and p is from 3 to 5. X and µ-X are preferably selected from the group consisting of RO⁻, Cl⁻, Br⁻, I⁻, F⁻, NCS⁻, N₃⁻, I₃⁻, NH₃, NR₃, RCOO⁻, RSO₃⁻, ROSO₃⁻, OH⁻, O²⁻, O₂²⁻, HOO⁻, H₂O, SH⁻, CN⁻, OCN⁻, C₂O₄²⁻ and SO₄²⁻, where R is alkyl, cycloalkyl, aryl or aralkyl with no more than 20 carbon atoms. Y is preferably selected from the group consisting of RO⁻, Cl⁻, Br⁻, I⁻, F⁻, RCOO-, SO₄²⁻, PF₆⁻, p-tolylsulfonate and trifluoromethylsulfonate, where R is alkyl, cycloalkyl, aryl or aralkyl with no more than 20 carbon atoms. Manganese may be in the oxidation state +2, +3, +4, or +7, the oxidation states +3 and +4 being preferred.

Preferred polydentate ligands are acyclic polyamines containing at least 7 atoms in the backbone or cyclic polyamines containing at least 9 atoms in the ring, each having the nitrogen atoms separated by at least two carbon atoms. Most preferred are ligands having a 1,4,7-triazacyclononane (Tacn) ring system, which may be substituted with one or more alkyl, cycloalkyl, aryl or aralkyl groups each containing up to 20 carbon atoms. Preferred substituents are methyl groups. Suitable ligands with a Tacn ring system are N',N",N"'-trimethyl-1,4,7-triazacyclononane (TmTacn) and 2-methyl-1,4,7-trimethyl-1,4,7-triazacyclononane, with TmTacn being preferred. Another suitable ligand is 1,5,9-trimethyl-1,5,9-triazacyclododecane.

Most preferred are the dinuclear manganese complexes [(TmTacn)Mn^{IV}(µ-O)₃Mn^{IV}(TmTacn)](PF₆)₂ and [(TmTacn)Mn^{IV}(µ-O)₃Mn^{IV}(TmTacn)](CH₃COO)₂.

The manganese complex may be formed in the reaction mixture by reaction of the polydentate ligand with a manganese salt, preferably manganese sulfate, manganese acetate, manganese nitrate, manganese chloride or manganese bromide with Mn²⁺ or Mn³⁺. Preferably, the manganese complex is prepared separately and added to the reaction mixture.

The water soluble epoxidation catalyst preferably comprises oxalic acid, an oxalate or a mixture of both as a co-catalyst in addition to the manganese complex. The co-catalyst is preferably used in a molar excess to the manganese complex, preferably with a molar ratio of co-catalyst to manganese complex in the range of from 10 : 1 to 10 000 : 1.

When a water soluble epoxidation catalyst is used, the organic phase may contain a water insoluble solvent, but preferably contains less than 30 % by weight, more preferably less than 5 % by weight of a solvent.

In another embodiment of the invention, the homogeneous epoxidation catalyst comprises a heteropolytungstate. The heteropolytungstate preferably comprises phosphorus or arsenic as heteroatom, most preferably phosphorus, i.e. the heteropolytungstate is a polytungstophosphate. Heteropolytungstates are known from the prior art. Most preferred are polytungstophosphates with a molar ratio of phosphorus to tungsten of from 1 : 2 to 1 : 12. The polytungstophosphate is preferably generated in situ from phosphoric acid and sodium tungstate, which are preferably employed in a molar ratio of phosphorus to tungsten of from 1 : 2 to 10 : 1. A polytungstophosphate reacts with hydrogen peroxide in the aqueous phase to give peroxotungstates and peroxotungstophosphates, such as PO₄[WO(O₂)₂]₄³⁻ and HPO₄[WO(O₂)₂]₂²⁻ and the corresponding partially protonated species.

The heteropolytungstate is preferably used in combination with a phase transfer catalyst. The term phase transfer catalyst refers to a compound comprising a cation or forming a cation in the aqueous phase, which cation forms a salt with the peroxotungstate and peroxotungstophosphate that is soluble in the organic phase. The phase transfer catalyst preferably comprises a singly charged cation or a compound forming a singly charged cation in the aqueous phase. Suitable as phase transfer catalysts are quaternary ammonium salts, tertiary amines and quaternary phosphonium salts. Suitable quaternary ammonium salts are tetraalkylammonium salts comprising at least 12 carbon atoms in total in the alkyl groups, such as dodecyltrimethylammonium salts, hexadecyltrimethylammonium salts, octadecyltrimethylammonium salts, methyltributylammonium salts and methyltrioctylammonium salts. Suitable quaternary ammonium salts may comprise singly and doubly charged anions, for example chloride, bromide, nitrate, sulfate, hydrogenphosphate, dihydrogenphosphate, methylsulfonate, methylsulfate and ethylsulfate. Suitable tertiary amines are dodecyldimethylamine, hexadecyldimethylamine, octadecyldimethylamine, tributylamine and trioctylamine.

The phase transfer catalyst is preferably used in an amount providing a molar ratio of phase transfer catalyst to tungsten of from 0.2 : 1 to 3 : 1, more preferably from 0.4 : 1 to 1 : 1, the molar ratio referring to the amount of cations or cation forming compounds in the phase transfer catalyst.

Preferably, the phase transfer catalyst comprises a salt with a quaternary ammonium ion of structure R¹R²R³R⁴N⁺, where R¹ is a group Y-O(C=O)R⁵, Y being a group CH₂CH₂, CH(CH₃)CH₂ or CH₂CH(CH₃) and R⁵ being an alkyl or alkenyl group containing 11 to 21 carbon atoms, R² is an alkyl group containing 1 to 4 carbon atoms and R³ and R⁴ are, independently of one another, R¹, R² or Y-OH. Preferred are salts with methyl sulfate as anion, for which R² is methyl and R⁵ is a linear alkyl or alkenyl group. More preferred are the salts (CH₃)₃N⁺CH₂CH₂O(C=O)R⁵ CH₃OSO₃⁻, (CH₃)₂N⁺(CH₂CH₂OH)(CH₂CH₂O(C=O)R⁵) CH₃OSO₃⁻, (CH₃)₂N⁺(CH₂CH₂O(C=O)R⁵)₂ CH₃OSO₃⁻, CH₃N⁺(CH₂CH₂OH)₂(CH₂CH₂O(C=O)R⁵) CH₃OSO₃⁻, CH₃N⁺(CH₂CH₂OH)(CH₂CH₂O(C=O)R⁵)₂ CH₃OSO₃⁻, CH₃N⁺(CH₂CH₂O(C=O)R⁵)₃ CH₃OSO₃⁻, (CH₃)₃N⁺CH₂CH(CH₃)O(C=O)R⁵ CH₃OSO₃⁻, (CH₃)₂N⁺(CH₂CH(CH₃)OH)(CH₂CH(CH₃)O(C=O)R⁵) CH₃OSO₃⁻ and (CH₃)₂N⁺(CH₂CH(CH₃)O(C=O)R⁵)₂ CH₃OSO₃⁻, in which R⁵ is a linear alkyl or alkenyl group containing 11 to 21 carbon atoms. Most preferred is the salt (CH₃)₂N⁺(CH₂CH(CH₃)O(C=O)R⁵)₂ CH₃OSO₃⁻ in which R⁵ is a linear alkyl or alkenyl group containing 11 to 17 carbon atoms. These preferred phase transfer catalysts can be prepared by esterifying ethanolamine, isopropanolamine, diethanolamine, diisopropanolamine, triethanolamine or triisopropanolamine with a fatty acid followed by quaternization with dimethylsulfate. Compared to tetraalkylammonium salts, these phase transfer catalysts have the advantage of being readily biodegradable which allows direct discharge of aqueous effluents from the method of the invention to a biological waste water treatment. Compared to tetraalkylammonium halides, these phase transfer catalysts provide reaction mixtures that are less corrosive. These preferred phase transfer catalysts are preferably added as a mixture comprising from 5 to 50 % by weight of a solvent selected from ethanol, 2-propanol, aliphatic hydrocarbons, fatty acids and fatty acid triglycerides to facilitate dosing and dispersing in the reaction mixture.

The heteropolytungstate and the phase transfer catalysts may be added as a mixture or separately, with separate addition to the reaction mixture being preferred.

When a heteropolytungstate is used in combination with a phase transfer catalysts, the resulting homogeneous epoxidation catalyst will be largely present in the organic liquid phase of the reaction mixture. With such a homogeneous epoxidation catalyst the epoxidation can be carried out both with and without addition of a solvent, which is preferably not water miscible. Preferably, an epoxidized fatty acid methyl ester is used as solvent. As an alternative to adding an epoxidized fatty acid methyl ester to the reaction mixture, the methyl ester of the corresponding unsaturated fatty acid may be added, which is then converted in the reaction mixture to the epoxidized fatty acid methyl ester. Preferred are epoxidized fatty acid methyl ester containing fatty acids derived from vegetable oils, preferably soy oil. Addition of an epoxidized fatty acid methyl ester prevents the formation of stable emulsions and facilitates phase separation of the two liquid phases of the reaction mixture. The solvent is preferably added in an amount providing a solvent content of the organic liquid phase of the reaction mixture of from 10 to 90 % by weight.

The reaction is carried out in a reaction mixture comprising an aqueous liquid phase and an organic liquid phase with mixing of the liquid phases. Preferably, the ratio of the volume of the aqueous phase to the volume of the organic phase is maintained in the range of from 10 : 1 to 1 : 10. When an epoxidation catalyst comprising a manganese complex is used, the ratio is more preferably from 2 : 1 to 1 : 4. Mixing of the liquid phases can be performed by turbulent flow of the reaction mixture, by passing reaction mixture through fixed mixing elements, such as static mixers, structured packings or random packings, or by a moving mixing element, such as a stirrer or a rotating pump.

Independently of which type of homogeneous epoxidation catalyst is used, the aqueous phase preferably comprises less than 30 % by weight, more preferably less than 5 % by weight of a water soluble solvent. The term solvent here refers to compounds added in addition to propene, epoxidation catalyst, co-catalyst or phase transfer catalyst, and impurities introduced with these components, such as propane introduced with propene, and does not encompass products formed from propene.

When an epoxidation catalyst comprising a manganese complex is used, the epoxidation reaction is preferably carried out at a temperature of from 0 °C to 70 °C, more preferably from 5 °C to 40 °C and most preferably from 10 °C to 30 °C. When an epoxidation catalyst comprising a heteropolytungstate is used, the epoxidation reaction is preferably carried out at a temperature of from 30 °C to 100 °C, more preferably from 60 °C to 90 °C.

The epoxidation is carried out at elevated pressure to maintain propene in the liquid phase. Epoxidation with a catalyst comprising a manganese complex is most preferably carried out at 10 °C to 20 °C and a pressure of from 0.8 to 2.0 MPa.. Epoxidation with a catalyst comprising a heteropolytungstate is most preferably carried out at 60 °C to 90 °C and a pressure of from 3 to 5 MPa.

The reaction is carried out continuously in a loop reactor. The term loop reactor here refers to a reactor in which reaction mixture is circulated driven by a pump. Pumping of the reaction mixture provides mixing of the liquid phases. The loop reactor may comprise vessels for increasing the volume in the loop and providing the residence time necessary for achieving the desired hydrogen peroxide conversion. Preferably, further mixing of the reaction mixture is provided in such vessels, for example by static mixers, structured packings or random packings arranged in a tube of enlarged diameter or by a stirred vessel arranged in the reactor loop. Preferably, a heat exchanger is arranged in the loop for cooling the reaction mixture in order to remove the heat of reaction, the reaction mixture preferably being passed through the heat exchanger in every cycle of the loop. The heat exchanger is preferably a tube bundle heat exchanger with the reaction mixture being passed through the tubes or a plate heat exchanger. The diameter of the tubes or the distance between plates is preferably chosen sufficiently narrow for providing turbulent flow and mixing of the two liquid phases.

The average residence time in the loop reactor, calculated as the ratio of the volume of the loop reactor divided by the sum of all fluid flows entering the loop reactor, is preferably selected to provide a hydrogen peroxide conversion of more than 85 %, more preferably of from 95 % to 99.5 %. For this purpose, the average residence time is preferably from 20 to 240 min.

Propene is preferably used in molar excess to hydrogen peroxide in order to achieve high conversion of hydrogen peroxide and the molar ratio of propene fed to the loop reactor to hydrogen peroxide fed to the loop reactor is preferably from 1.2 : 1 to 12 : 1, more preferably from 2 : 1 to 8 : 1. Propene can be used as a technical mixture with propane, preferably containing from 0.1 to 15 vol-% of propane.

When an epoxidation catalyst comprising a manganese complex is used, the amount of catalyst fed to the loop reactor is preferably chosen to provide a molar ratio of hydrogen peroxide fed to the loop reactor to manganese fed to the loop reactor of from 100 : 1 to 10 000 000 : 1, more preferably from 1000 : 1 to 1 000 000 : 1 and most preferably 10 000 : 1 to 100 000 : 1. When an epoxidation catalyst comprising a heteropolytungstate is used, the amount of catalyst fed to the loop reactor is preferably chosen to provide a molar ratio of hydrogen peroxide fed to the loop reactor to tungsten fed to the loop reactor of from 10 : 1 to 10 000 : 1, more preferably from 50 : 1 to 5 000 : 1.

When an epoxidation catalyst comprising a manganese complex is used, the concentration of hydrogen peroxide in the aqueous liquid phase is preferably maintained at less than 1.0 % by weight during the reaction. More preferably, the concentration of hydrogen peroxide is maintained at from 0.1 to 1.0 % by weight, most preferably at from 0.2 to 0.7 % by weight. When an epoxidation catalyst comprising a heteropolytungstate is used, the concentration of hydrogen peroxide in the aqueous liquid phase is preferably maintained at from 0.1 to 5 % by weight, preferably 0.5 to 3 % by weight. The concentration of hydrogen peroxide in the aqueous liquid phase may be adjusted by adjusting the molar ratio of propene to hydrogen peroxide fed to the loop reactor, adjusting the feed rate for feeding hydrogen peroxide to the loop reactor or adjusting the feed rate for feeding epoxidation catalyst to the reactor, with a higher molar ratio of propene to hydrogen peroxide, a lower feed rate for hydrogen peroxide or a higher feed rate for epoxidation catalyst leading to a lower concentration of hydrogen peroxide in the aqueous liquid phase.

In the method of the invention, aqueous phase is withdrawn from the loop reactor and extracted by liquid-liquid-extraction using propene as extractant. The extraction provides an extract comprising propene and propene oxide and an extracted aqueous phase depleted in propene oxide.

Preferably, reaction mixture is continuously withdrawn from the loop reactor, the withdrawn reaction mixture is separated into a separated aqueous phase and a separated organic phase and a part or all of the separated aqueous phase is extracted. The amount of withdrawn reaction mixture preferably corresponds to the total of liquid flows entering the mixed reactor in order to maintain a constant hold-up in the mixed reactor. Separation of the withdrawn reaction mixture into a separated aqueous phase and a separated organic phase may be carried out with any phase separating device known from the prior art. The phase separating device may be a settler or a device which applies a centrifugal force to the reaction mixture, such as a hydrocyclone or a centrifuge. The pressure in the phase separation is maintained higher than the vapor pressure of propene in order to maintain propene in the liquid phase.

The liquid-liquid-extraction may be carried out with any liquid-liquid-extraction device known from the prior art, such as a mixer/settler combination, a series of mixers and settlers, an extraction column or a centrifugal extractor. Preferably, the liquid-liquid-extraction is carried out in an extraction column operated in counter current flow. The extraction column may comprise trays, such as sieve trays, or may comprise packings, which may be structured packings or random packings. The internals of the extraction column preferably provide a separation efficiency of from 1 to 10 equilibrium stages, more preferably 2 to 5 equilibrium stages.

Liquid-liquid-extraction is preferably carried out at a temperature of from 0 to 40 °C, more preferably from 10 to 30 °C. When the epoxidation is carried out at a higher temperature, the reaction mixture withdrawn from the loop reactor or the aqueous phase passed to the liquid-liquid-extraction is preferably cooled in a heat exchanger to a temperature within this range. The pressure in the liquid-liquid-extraction is maintained higher than the vapor pressure of propene at the extraction temperature in order to maintain a liquid propene phase.

A part of the aqueous phase withdrawn from the loop reactor is continuously recycled into the loop reactor. The fraction of separated aqueous phase recycled into the loop reactor is preferably adjusted to maintain a constant phase ratio of aqueous liquid phase to organic liquid phase within the loop reactor. The part of the aqueous phase to be recycled into the loop reactor may be withdrawn before the liquid-liquid-extraction, passing the remaining aqueous phase to the liquid-liquid-extraction or it may be withdrawn after the liquid-liquid-extraction, recycling a part of the extracted aqueous phase into the loop reactor. Preferably, the aqueous phase to be recycled into the loop reactor is withdrawn before the liquid-liquid-extraction to reduce the flow of aqueous phase in the liquid-liquid-extraction.

The propene contained in the extract of the liquid-liquid-extraction is preferably passed to the loop reactor, preferably in the liquid state. For this purpose, the extract may be passed to a work-up stage in which propene is separated from propene oxide, for example by distillation, and the separated propene is passed to the loop reactor. This may involve combining the extract with separated organic phase obtained from phase separation of withdrawn reaction mixture, preferably before separating propene from propene oxide. However, in a preferred embodiment the extract is passed to the loop reactor without separating propene oxide and most preferably, the extract is passed directly to the loop reactor without any further separation step.

Propene starting material may be used as extractant. Alternatively or in addition to propene starting material, non-reacted propene recovered from the reaction mixture may be used as extractant. Preferably, the extractant comprises propene separated from an organic phase withdrawn from the loop reactor. Most preferably, all propene starting material and non-reacted propene recovered from the reaction mixture is passed to the liquid-liquid-extraction as extractant and the extract from the liquid-liquid-extraction is passed directly to the loop reactor without any further separation step.

When a propene starting material is used which comprises propane and non-reacted propene is recovered and recycled to the loop reactor, propane will accumulate in the loop reactor. In this case, a part of the recovered non-reacted propene is preferably withdrawn and subjected to a distillation in a C3-splitter column providing a bottom product enriched in propane, which is discharged, and an overhead product depleted in propane, which is recycled to the loop reactor.

When a water soluble epoxidation catalyst comprising a manganese complex is used and liquid-liquid-extraction is carried out in an extraction column, the catalyst contained in the aqueous phase fed to the extraction column will effect reaction of propene of the extractant with non-reacted hydrogen peroxide contained in the aqueous phase. The extraction column will then serve as an additional reactor for increasing hydrogen peroxide conversion to propene oxide. The extraction column may then be cooled, for example using one or several heat exchangers arranged within the extraction column, to remove the heat generated by this reaction.

In an alternative embodiment, reaction mixture comprising the manganese complex is withdrawn from the loop reactor and acid is added to the withdrawn reaction mixture before separating it into an organic phase A and an aqueous phase B. The acid is added in an amount to adjust the pH of aqueous phase B to a value between 1 and 2.5. Aqueous phase B is then extracted with propene in the liquid-liquid-extraction and part or all of the aqueous phase obtained after extraction is recycled to the loop reactor. Lowering of the pH reduces the catalytic activity of the manganese complex and at the same time increases its stability. As a result, further reaction of hydrogen peroxide with propene in the liquid-liquid-extraction is suppressed and the aqueous phase obtained after extraction comprises a higher fraction of catalytically active manganese complex which can be reused in the loop reactor upon recycling of the extracted aqueous phase.

Fig. 1 shows a preferred embodiment of the method of the invention using both propene starting material and non-reacted propene recovered from the reaction mixture as extractant in an extraction column operated in counter current flow.

Fig. 1 shows a loop reactor comprising a circulation pump (1), a heat exchanger (2) for cooling the reaction mixture, and a vessel (3) with a packing for keeping the aqueous phase and the organic phase mixed during the necessary retention time in the loop. Catalyst (4), comprising a water soluble manganese complex, and hydrogen peroxide (5) are fed to the loop reactor. Buffer and co-catalyst can also be fed to the loop reactor, but are not shown. Reaction mixture (6) is withdrawn from the loop reactor in an amount corresponding to the feeds to the loop reactor. The withdrawn reaction mixture comprises an organic phase, comprising propene oxide formed by the reaction and non-reacted propene, and an aqueous phase, comprising hydrogen peroxide, water formed from and introduced with the hydrogen peroxide, epoxidation catalyst and optional additives, such as buffer and co-catalyst. Withdrawn reaction mixture (6) is separated in a settler (7) into a separated aqueous phase (8) and a separated organic phase (9). A fraction (10) of the separated aqueous phase (8) is recycled into the loop reactor to maintain a constant phase ratio of aqueous liquid phase to organic liquid phase within the loop reactor and the remainder (11) is passed to an extraction column (12) operated in counter current. The separated organic phase (9) is passed to a first distillation column (13) where a stream (14) of non-reacted propene is separated from a crude propene oxide product (15). The stream (14) of non-reacted propene is combined with the propene starting material (16) and the combined stream (17) is fed as extractant to the extraction column (12). The extract (18) withdrawn from extraction column (12), comprising propene and propene oxide, is passed directly to the loop reactor as propene feed to the reaction. The extracted aqueous phase (19) is further processed for recovering propene and optionally 1,2-propanediol formed as a by-product (not shown).

The crude propene oxide product (15) is purified in a second distillation column (20) to provide a propene oxide overhead product (21) and a bottoms product (22) comprising high boiling byproducts. A stream comprising hydrazine (23) is preferably fed to the second distillation column above the feeding point of the crude propene oxide product (15) to remove carbonyl compounds comprised in the crude propene oxide.

A part of the stream (14) of non-reacted propene is withdrawn and fed to a C3-splitter column (24) where propane introduced with the propene starting material (16) is separated and withdrawn as bottoms product (25). The overhead product (26) from the C3-splitter column (24), which is depleted in propane, is returned to stream (14).

## Claims

1. A method for the epoxidation of propene, comprising continuously reacting propene with hydrogen peroxide in the presence of a homogeneous epoxidation catalyst, the reaction being carried out in a reaction mixture comprising an aqueous liquid phase and an organic liquid phase using a loop reactor with mixing of the liquid phases, **characterized in that** aqueous phase withdrawn from the loop reactor is extracted by liquid-liquid-extraction using propene as extractant to provide an extract comprising propene and propene oxide.

2. The method of claim 1, **characterized in that** reaction mixture is continuously withdrawn from the loop reactor, the withdrawn reaction mixture is separated into a separated aqueous phase and a separated organic phase and a part or all of the separated aqueous phase is extracted.

3. The method of claim 1 or 2, **characterized in that** the aqueous liquid phase of the reaction mixture comprises less than 30 % by weight of water soluble solvents.

4. The method of any one of claims 1 to 3, **characterized in that** liquid-liquid-extraction is carried out in an extraction column operated in counter current flow.

5. The method of any one of claims 1 to 4, **characterized in that** the propene contained in the extract is passed to the loop reactor.

6. The method of claim 5, **characterized in that** the extract is passed to the loop reactor without separating propene oxide.

7. The method of claim 5 or 6, **characterized in that** the extractant comprises all or a part of the propene starting material.

8. The method of any one of claims 1 to 7, **characterized in that** the extractant comprises propene separated from an organic phase withdrawn from the loop reactor.

9. The method of any one of claims 1 to 8, **characterized in that** the epoxidation catalyst comprises a water soluble manganese complex.

10. The method of claim 9, **characterized in that** the manganese complex carries a 1,4,7-trimethyl-1,4,7-triazacyclonane ligand.

11. The method of claim 9 or 10, **characterized in that** reaction mixture is withdrawn from the loop reactor and separated into an organic phase A and an aqueous phase B, acid is added to the withdrawn reaction mixture before separating phases in an amount to adjust the pH of aqueous phase B to a value between 1 and 2.5, aqueous phase B is extracted with propene and part or all of the aqueous phase obtained after extraction is recycled to the loop reactor.

12. The method of any one of claims 1 to 8, **characterized in that** the epoxidation catalyst comprises a heteropolytungstate.
